# FASCICULE DE BREVET EUROPEEN

(11) **EP 0 884 305 B1**
(45) Date de publication et mention de la délivrance du brevet: **13.06.2001**
(21) Numéro de dépôt: 98401158.5
(22) Date de dépôt: 14.05.1998
(51) Int. Cl.: C07C 231/02, C07C 233/18, C07C 235/08

(54) **Procédé de préparation de composés de type céramides**
Verfahren zur Herstellung von Ceramidverbindungen
Process for the preparation of ceramide compounds

(30) Priorité: 11.06.1997 FR 9707240
(43) Date de publication de la demande: 16.12.1998
(73) Titulaire: L'OREAL, 75008 Paris (FR)
(72) Inventeur: Semeria, Didier, 77181 Courtry (FR); Philippe, Michel, 91320 Wissous (FR)
(74) Mandataire: Dodin, Catherine

(56) Documents cités:
- M.P.VAZQUEZ-TATO: "Microwave-mediated synthesis of amides" SYNLETT., no. 7, 1993, page 506 XP002056049
- B. W. BALDWIN ET AL: "Improved microwave oven synthesis of amides amd imides promoted by imidazole; convenient transport agent preparation" CHEMICAL COMMUNICATIONS, no. 23, 1996, pages 2669-2670, XP002056050
- B. OUSSAID ET AL: "Improved synthesis of oxazoline under microwave irradiation" SYNTHETIC COMMUNICATIONS, vol. 25, no. 5, 1995, pages 659-665, XP002056287
- A. L. MARRERO-TERRERO ET AL: "Synthesis of 2-oxazolines from carboxylic acids and alpha,alpha,alpha-tris(hydroxymethyl)methy lamine under microwaves in solvent-free conditions" SYNLETT,mars 1996, pages 245-246, XP002056051
- DATABASE WPI Week 9816 Derwent Publications Ltd., London, GB; AN 98-174845 XP002076916 "Preparation of acid amide, acid imide or ester compounds - by dehydration condensation of organic carboxylic acid with amine or alcohol under microwave conditions" & JP 10 036294 A (AGENCY OF IND. SCI. & TECHNOLOGY), 10 février 1998

## Description

L'invention a pour objet un nouveau procédé de préparation de composés de type céramides.

Les céramides, à l'état naturel, sont les composants principaux des couches lipidiques de l'épiderme. Ils sont utilisés en cosmétique sous forme naturelle ou synthétique dans des compositions destinées, entre autre, à réduire le dessèchement de la peau ou à conférer à celle-ci une meilleure élasticité, ou encore destinées au traitement du cheveu.

Les céramides naturels sont généralement obtenus par extraction de la peau de porc, du cerveau de boeuf, de l'oeuf, des cellules du sang, des plantes (JP 86-260008 ou JP 86-120308).

Les inconvénients liés à ce type d'approvisionnement (fragilité, contamination, conservation, coût,) ont fait que très tôt la voie de synthèse chimique a été explorée.

Des céramides de synthèse ont ainsi déjà été proposés dans la demande EP-A-500 437 ou EP 0 647 617. Ces céramides résultent de l'acylation de la fonction amine d'une sphingosine ou d'une dihydrosphingosine par un acide activé, conduisant ainsi à des composés comportant une fonction amide. Les réactions d'acylation peuvent être effectuées par de nombreuses méthodes qui sont décrites par J. March dans Advanced Organic Chemistry - Fourth Edition - JOHN WILEY & SONS, INC, p 417-425 (1992). L'acide activé utilisé pour la réaction d'acylation peut être par exemple un chlorure d'acide, un ester, un anhydride, un azolide et doit être synthétisé avant d'effectuer la réaction d'acylation conduisant à la formation de la fonction amide du céramide. En outre, si l'acide sous sa forme non activée comporte un ou plusieurs groupements hydroxyles autres que celui du groupement acide carboxylique, ce ou ces groupements hydroxyles doivent être nécessairement protégés pour ne pas réagir lors de la synthèse visant à former la liaison amide. Cette voie de synthèse nécessite donc une étape de synthèse préalable d'activation de l'acide.

Il est aussi connu par Mitchell, Reid dans J. Am. Chem. Soc., vol 53, p 1879 (1931) que la pyrolyse de sels d'amine et d'acide peut conduire à la formation de liaison amide mais cette méthode nécessite plusieurs heures de chauffage entre 160 °C et 220 °C. La température élevée nécessaire à la réaction provoque des décompositions importantes des produits de départ ou des produits formés conduisant ainsi à de faibles rendements en produits synthétisés. Par ailleurs, lorsque l'on utilise des amino-alcools comme produits de départs, ces réactions de pyrolyse conduisent de façon majoritaire à des dérivés de type oxazoline au lieu des céramides souhaités.

L'utilisation des micro-ondes au cours de réactions chimiques d'amines avec des acides pour produire des amides a été décrite par P. Vazquez-Tato dans Synlett, 1993, p 506 . La publication de B. Oussaid, Synthetic Communications, 25(5), p 659-665 (1995) montre que ce type de réaction réalisée avec l'acide 2-thiényl acétique et l'éthanolamine conduit à un mélange d'amide et d'ester , avec un rendement en amide de 40 %. A. L. Marrero-Terrero, Synlett, p 245 (1996) montre que la réaction conduite avec l'α,α,α-tris (hydroxyméthyl)méthylamine ne produit pas d'amide mais une oxazoline.

Le but de la présente invention est de proposer un nouveau procédé de préparation de composés de type céramides ne nécessitant pas d'utiliser d'acide activé et conduisant directement au produit souhaité avec un rendement élevé, et notamment avec un rendement de plus de 60 %. L'invention a aussi pour but de proposer un procédé de préparation de composés de type céramides ne nécessitant pas de protéger les fonctions hydroxyles éventuellement présentes dans les produits de départ.

On a découvert que la réaction d'amino-alcools particuliers avec un acide à l'aide de micro-ondes permet de synthétiser des composés de type céramides de manière très rapide tout en obtenant de bons rendements en minimisant la formation de produits secondaires, en particulier les esters et les oxazolines.

Ainsi, l'objet de la présente invention a pour objet un procédé de préparation de composés de type céramides de formule (I) 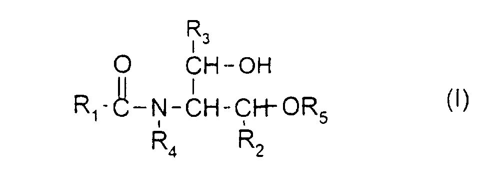 , caractérisé par le fait que l'on fait réagir en une seule étape un amino-alcool de formule (II) 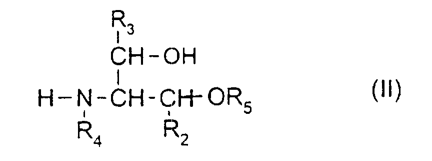 en présence d'un acide de formule (III) R₁-COOH, formules dans lesquelles :
- R₁ désigne :
   - (i) soit un radical hydrocarboné, linéaire ou ramifié, saturé ou insaturé, en C₁-C₅₀, de préférence en C₅-C₅₀, et plus préférentiellement en C₇-C₃₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ et de préférence en C₁₆-C₃₀, le ou les hydroxyles du radical R₇ pouvant être estérifiés par un acide gras saturé ou insaturé, linéaire ou ramifié, éventuellement mono ou polyhydroxylé, en C₁-C₃₅,
   - (ii) soit un radical R"-(NR-CO)-R', R désigne un atome d'hydrogène ou un radical hydrocarboné C₁-C₂₀, de préférence C₁-C₁₀, mono ou polyhydroxylé, préférentiellement monohydroxylé, R' et R" sont des radicaux hydrocarbonés dont la somme des atomes de carbone est comprise entre 9 et 30, R' étant un radical divalent,
   - (iii) soit un radical R₈-O-CO-(CH2)ₚ, R₈ désignant un radical hydrocarboné en C₁-C₂₀, p étant un entier variant de 1 à 12 ;
- R₂ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)n, (galactosyle)ₘ sulfogalactosyle, phosphoryléthylamine ou phosphoryléthylammonium, dans lesquels n est un entier variant de 1 à 4 et m est un entier variant de 1 à 8 ; de préférence, R₂ désigne un radical hydrocarboné en C₁-C₄ saturé ou insaturé, linéaire ou ramifié
- R₃ désigne un radical hydrocarboné en C₁-C₃₂, de préférence C₁₀-C₂₅, saturé ou insaturé, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ et de préférence en C₁₆-C₃₀, R3 pouvant également être substitué par un ou plusieurs radicaux alkyle en C₁-C₁₄ ;
- R₄ désigne un atome d'hydrogène, un radical méthyle, éthyle, un radical hydrocarboné en C₃-C₅₀, de préférence en C₁₆-C₂₇, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p étant un entier variant de 1 à 12,
- R₅ désigne un atome d'hydrogène ou un radical de type saccharidique, en particulier un radical (glycosyle)n, (galactosyle)ₘ, ou sulfogalactosyle, dans lesquels n est un entier variant de 1 à 4 et m est un entier variant de 1 à 8; de préférence, R₅ désigne un atome d'hydrogène,
la réaction étant effectuée par irradiation de micro-ondes et à une température inférieure ou égale à 180 °C. Avantageusement, la température de la réaction est supérieure ou égale à 100 °C.

De préférence, la réaction est effectuée à une température supérieure ou égale à 110 °C, et préférentiellement entre 120 °C et 180 °C.

Par micro-ondes, on entend dans la présente invention les ondes Ultra-Hautes Fréquences dont la fréquence va de 300 MHz à 30 GHz. On peut utiliser plus particulièrement les ondes dont la fréquence va de 800 MHz à 3000 MHz et de préférence de 2400 MHz à 2500 MHz.

De préférence, pour les composés de formule (I) à (III), R₁ désigne un radical tel que défini selon (i), et en particulier un radical alkyle en C₁-C₃₁ , linéaire ou ramifié, saturé ou insaturé, hydroxylé ou non ; R₂ désigne un atome d'hydrogène ; R₃ désigne un radical alkyle en C₁₀-C₂₅, linéaire ou ramifié, saturé ou insaturé, hydroxylé ou non ; R₄ désigne un atome d'hydrogène ; R₅ désigne un atome d'hydrogène.

Lorsque l'on effectue le mélange de l'amine de formule (II) et l'acide de formule (III) tels que définis précédemment, il se forme en premier lieu le carboxylate d'ammonium correspondant, généré par l'échange d'un proton entre ces deux produits de départ. Dans une variante de réalisation du procédé selon l'invention, il est possible de préparer préalablement le carboxylate d'ammonium, puis de procéder à son irradiation à l'aide de micro-ondes.

Le temps d'irradiation des micro-ondes pendant la réaction selon le procédé de l'invention peut être très court, c'est-à-dire de l'ordre de 5 secondes, ou bien être plus long, par exemple jusqu'à environ 4 heures. De préférence, le mélange réactionnel peut être irradié pendant une période allant de 1 minute à 2 heures.

La réaction peut être effectuée en présence de solvant qui sera choisi en particulier parmi les solvants ayant un point d'ébullition allant de 100 °C à 220 °C. Avantageusement, la réaction est conduite en l'absence de solvant, ce qui évite une étape d'élimination du solvant une fois la réaction terminée.

Bien que la réaction en présence de micro-ondes s'effectue très rapidement, il est possible d'accélérer le temps de la réaction en éliminant l'eau formée au cours de la réaction. L'eau formée peut être éliminée soit en réduisant la pression du milieu de réaction, soit en ajoutant au milieu de réaction un catalyseur de déshydratation. La pression du milieu réactionnel peut être réduite à une pression inférieure à 10⁵ Pa. Comme catalyseur de déshydratation, on peut notamment utiliser l'acide pyrosulfurique, l'acide phosphorique, l'acide paratoluène sulfonique, ou bien encore des argiles telles que les montmorillonites.

Pour mettre en oeuvre le procédé selon l'invention, on effectue le mélange de l'amine de formule (II) et de l'acide de formule (III) et on irradie le mélange à l'aide de micro-ondes générés par un four à micro-ondes. La puissance du four à micro-ondes peut aller de 1 W à 2000 W, et plus particulièrement de 1 W à 600 W.

Lorsque la réaction est terminée, les produits obtenus peuvent être isolés du milieu réactionnel par tout procédé bien connu de l'homme du métier.

On va donner maintenant, sans effet limitatif, des exemples de synthèse selon l'invention.

### Exemple 1 :

### Préparation du 2-(2'-hydroxy hexadécanoyl)-amino octadécane-1,3-diol

4,9 g (0,018 mole) d'acide 2-hydroxyhexadécanoïque et 5,4 g (0,018 mole) de 2-amino octadécane-1,3-diol ont été mélangés dans un tube de verre ; le mélange a été irradié par des micro-ondes à l'aide d'un appareil SYNTHEWAVE 402 ™ de PROLABO - fréquence 2450 ± 50 MHz - puissance modulable 300 W. Après une irradiation de 15 minutes à 155 °C ± 5 °C, le mélange réactionnel a été solubilisé à chaud dans un mélange de 80 ml d'acétate d'éthyle et 20 ml d'heptane. Le précipité obtenu a été recristallisé dans l'éthanol et on a obtenu 7 g de produit pur attendu, avec un rendement de 70 %.
Point de fusion : 88 °C
Le spectre RMN ¹³C est conforme à la structure attendue.

### Exemple 2 :

### Préparation du 2-octadécanoylamino octadécane-1,3-diol

6,2 g (0,022 mole) d'acide stéarique et 6 g (0,022 mole) de 2-amino octadécane-1,3-diol ont été mélangés dans un tube et irradiés avec l'appareil utilisé dans l'exemple 1, dans les mêmes conditions de fréquence et de puissance. Après une irradiation de 18 minutes à 140 °C ± 5 °C, le mélange réactionnel a été solubilisé dans un mélange de 80 ml d'acétate d'éthyle et 40 ml d'heptane. Le précipité obtenu a été recristallisé dans l'éthanol et on a obtenu 8,1 g de produit pur attendu, avec un rendement de 72 %.
Point de fusion : 93 - 101 °C
Le spectre RMN ¹³C est conforme à la structure attendue.

## Revendications

1. Procédé de préparation de composés de type céramides de formule (I) 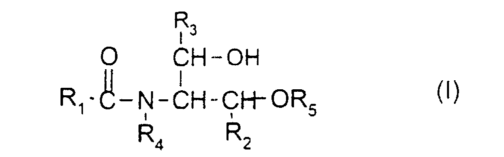 , caractérisé par le fait que l'on fait réagir en une seule étape un amino-alcool de formule (II) 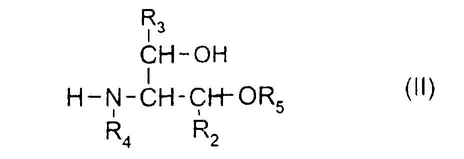 en présence d'un acide de formule (III) R₁-COOH, formules dans lesquelles :
- R₁ désigne :
- soit un radical hydrocarboné, linéaire ou ramifié, saturé ou insaturé, en C₁-C₅₀, de préférence en C₅-C₅₀, et plus préférentiellement en C₇-C₃₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ et de préférence en C₁₆-C₃₀, le ou les hydroxyles du radical R₇ pouvant être estérifiés par un acide gras saturé ou insaturé, linéaire ou ramifié, éventuellement mono ou polyhydroxylé, en C₁-C₃₅,
- soit un radical R"-(NR-CO)-R', R désigne un atome d'hydrogène ou un radical hydrocarboné C₁-C₂₀, de préférence C₁-C₁₀, mono ou polyhydroxylé, préférentiellement monohydroxylé, R' et R" sont des radicaux hydrocarbonés dont la somme des atomes de carbone est comprise entre 9 et 30, R' étant un radical divalent,
- soit un radical R₈-O-CO-(CH₂)ₚ, R₈ désignant un radical hydrocarboné en C₁-C₂₀, p étant un entier variant de 1 à 12 ;
- R₂ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)_{m,} sulfogalactosyle, phosphoryléthylamine ou phosphoryléthylammonium, dans lesquels n est un entier variant de 1 à 4 et m est un entier variant de 1 à 8; de préférence, R₂ désigne un radical hydrocarboné en C₁-C₄ saturé ou insaturé, linéaire ou ramifié
- R₃ désigne un radical hydrocarboné en C₁-C₃₂, de préférence C₁₀-C₂₅, saturé ou insaturé, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ et de préférence en C₁₆-C₃₀, R₃ pouvant également être substitué par un ou plusieurs radicaux alkyle en C₁-C₁₄ ;
- R₄ désigne un atome d'hydrogène, un radical méthyle, éthyle, un radical hydrocarboné en C₃-C₅₀, de préférence en C₁₆-C₂₇, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p étant un entier variant de 1 à 12,
- R₅ désigne un atome d'hydrogène ou un radical de type saccharidique, en particulier un radical (glycosyle)n, (galactosyle)_{m,} ou sulfogalactosyle, dans lesquels n est un entier variant de 1 à 4 et m est un entier variant de 1 à 8; de préférence, R₅ désigne un atome d'hydrogène,
la réaction étant effectuée par irradiation de micro-ondes et à une température inférieure ou égale à 180 °C.

2. Procédé selon la revendication 1, caractérisé par le fait que la température de réaction est supérieure ou égale à 100 °C.

3. Procédé selon la revendication 1 ou 2, caractérisé par le fait que la température de réaction va de 110 °C à 180 °C, et mieux de 120 °C à 180 °C.

4. Procédé selon l'une quelconque des revendications précédentes, caractérisé par le fait que R₁ désigne un radical alkyle en C₁-C₃₁, linéaire ou ramifié, saturé ou insaturé, hydroxylé ou non ; R₂ désigne un atome d'hydrogène ; R₃ désigne un radical alkyle en C₁₀-C₂₅, linéaire ou ramifié, saturé ou insaturé, hydroxylé ou non ; R₄ désigne un atome d'hydrogène ; R₅ désigne un atome d'hydrogène.

5. Procédé selon l'une quelconque des revendications précédentes, caractérisé par le fait que les micro-ondes ont une fréquence allant de 300 MHz à 30 GHz.

6. Procédé selon la revendication 5, caractérisé par le fait que les micro-ondes ont une fréquence allant de 800 MHz à 3000 MHz.

7. Procédé selon l'une quelconque des revendications précédentes, caractérisé par le fait que les micro-ondes ont une fréquence allant de 2400 MHz à 2500 MHz.

8. Procédé selon l'une quelconque des revendications précédentes, caractérisé par le fait que la réaction est effectuée en présence de solvant .

9. Procédé selon l'une quelconque des revendications 1 à 7, caractérisé par le fait que la réaction est effectuée en l'absence de solvant.

10. Procédé selon l'une quelconque des revendications précédentes, caractérisé par le fait que la réaction est effectuée en présence d'un catalyseur de déshydratation.

11. Procédé selon l'une quelconque des revendications précédentes, caractérisé par le fait que le mélange réactionnel est irradié par les micro-ondes pendant une période allant de 5 secondes à 4 heures.

## Claims

1. Process for the preparation of ceramide-type compounds of formula (I) 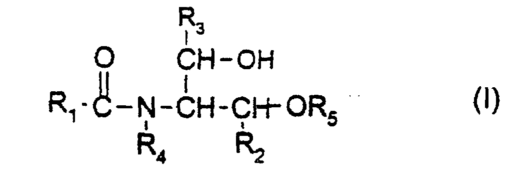 characterized in that an amino alcohol of formula (II) 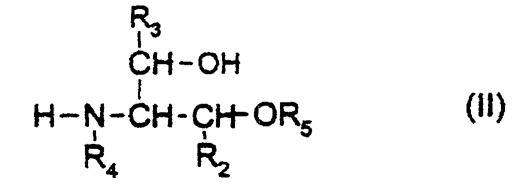 is reacted, in a single step, in the presence of an acid of formula (III) R₁-COOH, in which formulae:
- R₁ denotes:
- either a saturated or unsaturated, linear or branched C₁-C₅₀, preferably C₅-C₅₀, and more preferably C₇-C₃₀, hydrocarbon radical, it being possible for this radical to be substituted with one or more hydroxyl groups optionally esterified with an acid R₇COOH, R₇ being an optionally mono- or polyhydroxylated, saturated or unsaturated, linear or branched C₁-C₃₅, preferably C₁₆-C₃₀, hydrocarbon radical, it being possible for the hydroxyl(s) of the R₇ radical to be esterified with an optionally mono- or polyhydroxylated, saturated or unsaturated, linear or branched C₁-C₃₅ fatty acid,
- or a radical R"-(NR-CO)-R', R denotes a hydrogen atom or a mono- or polyhydroxylated, preferably monohydroxylated, C₁-C₂₀, preferably C₁-C₁₀, hydrocarbon radical, R' and R" are hydrocarbon radicals in which the sum of the carbon atoms is between 9 and 30, R' being a divalent radical,
- or a radical R₈-O-CO-(CH₂)ₚ, R₈ denoting a C₁-C₂₀ hydrocarbon radical, p being an integer varying from 1 to 12;
- R₂ denotes a hydrogen atom or an optionally mono- or polyhydroxylated, saturated or unsaturated, linear or branched C₁-C₃₀ hydrocarbon radical, it being possible for the hydroxyl(s) to be etherified with a (glycosyl)ₙ, (galactosyl)ₘ, sulphogalactosyl, phosphorylethylamine or phosphorylethylammonium radical, in which n is an integer varying from 1 to 4 and m is an integer varying from 1 to 8; preferably, R₂ denotes a saturated or unsaturated, linear or branched C₁-C₄ hydrocarbon radical;
- R₃ denotes a saturated or unsaturated C₁-C₃₂, preferably C₁₀-C₂₅, hydrocarbon radical, it being possible for this radical to be substituted with one or more hydroxyl groups optionally esterified with an acid R₇COOH, R₇ being an optionally mono- or polyhydroxylated, saturated or unsaturated, linear or branched C₁-C₃₅, and preferably C₁₆-C₃₀, hydrocarbon radical, it being also possible for R3 to be substituted with one or more C₁-C₁₄ alkyl radicals;
- R₄ denotes a hydrogen atom, a methyl radical, an ethyl radical, an optionally hydroxylated, saturated or unsaturated, linear or branched C₃-C₅₀, preferably C₁₆-C₂₇, hydrocarbon radical or a radical -CH₂-CHOH-CH₂-O-R₆ in which R₆ denotes a C₁₀-C₂₆ hydrocarbon radical or a radical R₈-O-CO-(CH₂)ₚ, R₈ denotes a C₁-C₂₀ hydrocarbon radical, p being an integer varying from 1 to 12;
- R₅ denotes a hydrogen atom or a saccharide-type radical, in particular a (glycosyl)ₙ, (galactosyl)ₘ or sulphogalactosyl radical in which n is an integer varying from 1 to 4 and m is an integer varying from 1 to 8; preferably, R₅ denotes a hydrogen atom; the reaction being carried out by irradiation with microwaves and at a temperature of less than or equal to 180°C.

2. Process according to Claim 1, characterized in that the reaction temperature is greater than or equal to 100°C.

3. Process according to Claim 1 or 2, characterized in that the reaction temperature ranges from 110°C to 180°C, and better still from 120°C to 180°C.

4. Process according to any-one of the preceding claims, characterized in that R₁ denotes a hydroxylated or nonhydroxylated, saturated or unsaturated, linear or branched C₁-C₃₁ alkyl radical; R₂ denotes a hydrogen atom; R₃ denotes a hydroxylated or nonhydroxylated, saturated or unsaturated, linear or branched C₁₀-C₂₅ alkyl radical; R₄ denotes a hydrogen atom; R₅ denotes a hydrogen atom.

5. Process according to any one of the preceding claims, characterized in that the microwaves have a frequency ranging from 300 MHz to 30 GHz.

6. Process according to Claim 5, characterized in that the microwaves have a frequency ranging from 800 MHz to 3000 MHz.

7. Process according to any one of the preceding claims, characterized in that the microwaves have a frequency ranging from 2400 MHz to 2500 MHz.

8. Process according to any one of the preceding claims, characterized in that the reaction is carried out in the presence of solvent.

9. Process according to any one of Claims 1 to 7, characterized in that the reaction is carried out in the absence of solvent.

10. Process according to any one of the preceding claims, characterized in that the reaction is carried out in the presence of a dehydration catalyst.

11. Process according to any one of the preceding claims, characterized in that the reaction mixture is irradiated with microwaves for a period ranging from 5 seconds to 4 hours.

## Patentansprüche

1. Verfahren zur Herstellung von Verbindungen vom Ceramid-Typ der Formel (I): 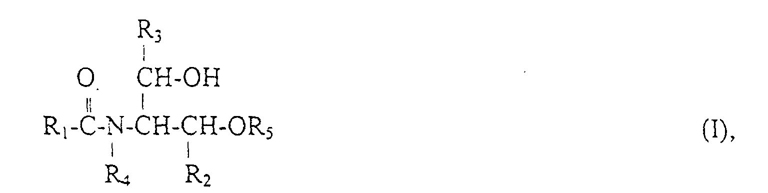 dadurch gekennzeichnet, daß in einem einzigen Schritt ein Aminoalkohol der Formel (II) 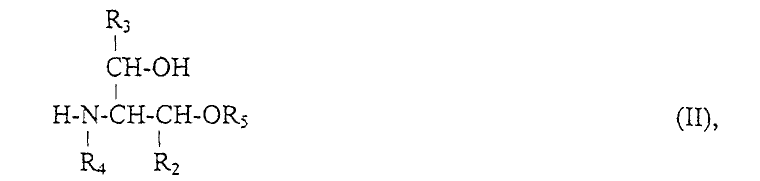 mit einer Säure der Formel (III) R₁-COOH umgesetzt wird, wobei in den Formeln bedeuten:
R₁
· eine geradkettige oder verzweigte, gesättigte oder ungesättigte Kohlenwasserstoffgruppe mit 1 bis 50, vorzugsweise 5 bis 50 und noch bevorzugter 7 bis 30 Kohlenstoffatomen, wobei die Gruppe mit einer oder mehreren Hydroxygruppen substituiert sein kann, die gegebenenfalls mit einer Säure R₇COOH verestert sind, worin die Gruppe R₇ eine gesättigte oder ungesättigte, geradkettige oder verzweigte, gegebenenfalls mono- oder polyhydroxylierte Kohlenwasserstoffgruppe mit 1 bis 35 und vorzugsweise 16 bis 30 Kohlenstoffatomen ist, wobei die Hydroxygruppe(n) der Gruppe R₇ mit einer gesättigten oder ungesättigten, geradkettigen oder verzweigten, gegebenenfalls mono- oder polyhydroxylierten C₁₋₃₅-Fettsäure verestert sein kann (können),
· eine Gruppe R"-(NR-CO)-R', wobei R ein Wasserstoffatom oder eine mono- oder polyhydroxylierte und vorzugsweise monohydroxylierte Kohlenwasserstoffgruppe mit 1 bis 20 und vorzugsweise 1 bis 10 Kohlenstoffatomen bedeutet und R' und R" Kohlenwasserstoffgruppen sind, wobei die Summe ihrer Kohlenstoffatome im Bereich von 9 bis 30 liegt und wobei R' eine zweiwertige Gruppe ist,
· eine Gruppe R₈-O-CO-(CH₂)ₚ, wobei R₈ eine C₁₋₂₀-Kohlenwasserstoffgruppe bedeutet und p eine ganze Zahl von 1 bis 12 ist;
R₂ ein Wasserstoffatom oder eine gesättigte oder ungesättigte, geradkettige oder verzweigte, gegebenenfalls mono- oder polyhydroxylierte Kohlenwasserstoffgruppe mit 1 bis 30 Kohlenstoffatomen, wobei die Hydroxygruppe(n) mit einer Gruppe (Glycosyl)ₙ, (Galactosyl)ₘ, Sulfogalactosyl, Phosphorylethylamin oder Phosphorylethylammonium verethert sein kann (können), wobei n eine ganze Zahl von 1 bis 4 und m eine ganze Zahl von 1 bis 8 bedeutet; R₂ bedeutet vorzugsweise eine gesättigte oder ungesättigte, geradkettige oder verzweigte C₁₋₄-Kohlenwasserstoffgruppe;
R₃ eine gesättigte oder ungesättigte Kohlenwasserstoffgruppe mit 1 bis 32 und vorzugsweise 10 bis 25 Kohlenstoffatomen, wobei die Gruppe mit einer oder mehreren Hydroxygruppen substituiert sein kann, die gegebenenfalls mit einer Säure R₇COOH verestert sind, worin die Gruppe R₇ eine gesättigte oder ungesättigte, geradkettige oder verzweigte, gegebenenfalls mono- oder polyhydroxylierte Kohlenwasserstoffgruppe mit 1 bis 35 und vorzugsweise 16 bis 30 Kohlenstoffatomen ist, wobei R₃ auch mit einer oder mehreren C₁₋₁₄-Alkylgruppen substituiert sein kann;
R₄ ein Wasserstoffatom, eine Methylgruppe, eine Ethylgruppe, eine gesättigte oder ungesättigte, geradkettige oder verzweigte, gegebenenfalls hydroxylierte Kohlenwasserstoffgruppe mit 3 bis 50 und vorzugsweise 16 bis 27 Kohlenstoffatomen, eine Gruppe -CH₂-CHOH-CH₂-O-R₆, wobei R₆ eine C₁₀₋₂₆-Kohlenwasserstoffgruppe bedeutet, oder eine Gruppe R₈-O-CO-(CH₂)ₚ, wobei R₈ eine C₁₋₂₀-Kohlenwasserstoffgruppe bedeutet und p eine ganze Zahl von 1 bis 12 ist;
R₅ ein Wasserstoffatom oder eine Gruppe vom Saccharidtyp, insbesondere (Glycosyl)ₙ, (Galactosyl)ₘ oder Sulfogalactosyl, wobei n eine ganze Zahl von 1 bis 4 und m eine ganze Zahl von 1 bis 8 bedeutet; R₅ bedeutet vorzugsweise ein Wasserstoffatom;
wobei die Umsetzung unter Mikrowellenbestrahlung bei einer Temperatur von höchstens 180 °C durchgerührt wird.

2. Verfahren nach Anspruch 1, dadurch gekennzeichnet, daß die Reaktionstemperatur mindestens 100 °C beträgt.

3. Verfahren nach Anspruch 1 oder 2, dadurch gekennzeichnet, daß die Reaktionstemperatur im Bereich von 110 bis 180 und besser noch 120 bis 180 °C liegt.

4. Verfahren nach einem der vorhergehenden Ansprüche, dadurch gekennzeichnet, daß bedeuten: R₁ eine gesättigte oder ungesättigte, geradkettige oder verzweigte, gegebenenfalls hydroxylierte C₁₋₃₁-Alkylgruppe; R₂ ein Wasserstoffatom; R₃ eine gesättigte oder ungesättigte, geradkettige oder verzweigte, gegebenenfalls hydroxylierte C₁₀₋₂₅-Alkylgruppe; R₄ ein Wasserstoffatom; und R₅ ein Wasserstoffatom.

5. Verfahren nach einem der vorhergehenden Ansprüche, dadurch gekennzeichnet, daß die Mikrowellen eine Frequenz von 300 MHz bis 60 GHz aufweisen.

6. Verfahren nach Anspruch 5, dadurch gekennzeichnet, daß die Mikrowellen eine Frequenz von 800 bis 3000 MHz aufweisen.

7. Verfahren nach einem der vorhergehenden Ansprüche, dadurch gekennzeichnet, daß die Mikrowellen eine Frequenz von 2400 bis 2500 MHz aufweisen.

8. Verfahren nach einem der vorhergehenden Ansprüche, dadurch gekennzeichnet, daß die Umsetzung in Gegenwart eines Lösungsmittel durchgeführt wird.

9. Verfahren nach einem der Ansprüche 1 bis 7, dadurch gekennzeichnet, daß die Umsetzung ohne Lösungsmittel durchgeführt wird.

10. Verfahren nach einem der vorhergehenden Ansprüche, dadurch gekennzeichnet, daß die Umsetzung in Gegenwart eines Dehydratisierungsmittels durchgeführt wird.

11. Verfahren nach einem der vorhergehenden Ansprüche, dadurch gekennzeichnet, daß das Reaktionsgemisch während einer Zeitspanne von 5 s bis 4 h mit Mikrowellen bestrahlt wird.
